# EUROPÄISCHE PATENTSCHRIFT

(11) **EP 3 528 701 B1**
(45) Veröffentlichungstag und Bekanntmachung des Hinweises auf die Patenterteilung: **24.12.2025**
(21) Anmeldenummer: 17791543.6
(22) Anmeldetag: 19.10.2017
(51) Int. Cl.: A61N 1/04, A61B 5/259, A61B 5/266, A61B 5/274, A61B 5/265, A61B 5/291

(54) **ELEKTRODE ZUM ANBRINGEN AUF DER MENSCHLICHEN HAUT**
ELECTRODE FOR APPLICATION TO HUMAN SKIN
ÉLECTRODE À APPLIQUER SUR LA PEAU HUMAINE

(30) Priorität: 21.10.2016 AT 509712016
(43) Veröffentlichungstag der Anmeldung: 28.08.2019
(73) Patentinhaber: Leonh. Lang, 6020 Innsbruck (AT)
(72) Erfinder: LANG, Burrhus, 6020 Innsbruck (AT)
(74) Vertreter: Torggler & Hofmann Patentanwälte - Innsbruck
(86) Internationale Anmeldenummer: PCT/AT2017/060276
(87) Internationale Veröffentlichungsnummer: WO 2018/071944

(56) Entgegenhaltungen:
- WO-A1-2006/053366
- WO-A1-2016/001393
- WO-A2-01/17423
- DE-U1- 202007 006 112
- US-A- 4 117 846
- US-A- 4 777 954

## Beschreibung

Die Erfindung betrifft eine Elektrode nach Oberbegriff des Anspruchs 1. Weiters betrifft die Erfindung ein Verfahren zum Herstellen einer Elektrode.

Derartige medizinische Hautelektroden können als Messelektroden eingesetzt werden, die elektrische Signale vom menschlichen Körper ableiten. Sie können aber auch als Therapieelektroden eingesetzt werden, um Ströme dem menschlichen Körper zuzuführen. Die Elektroden werden zu diesem Zweck auf die Haut aufgeklebt und verfügen auf ihrer Unterseite im Allgemeinen ein elektrisch leitendes Gel oder ein anderes elektrisches Kontaktmedium, das galvanisch mit einem Anschlusselement der Elektrode in Kontakt steht. An diesem Anschlusselement kann ein elektrischer Signalleiter angeschlossen werden, über den Ströme aus der Elektrode abgeleitet oder der Elektrode zugeführt werden können.

Ein Typ von Elektroden weist an der der Haut abgewandten Oberseite ein vorgestehendes elektrisch leitendes Anschlusselement mit einer meist im wesentlichen kugelkopfförmigen Anschlussstelle auf, an die sich ein Hals anschließt.

Bei der bisherigen Konstruktion von Elektroden dieses Typs war das Anschlusselement zweiteilig ausgeführt. Der obere Teil (Oberknopf, Stud) dient als Kontakt- und Ankerelement für handelsübliche Signalleiter, beispielsweise EKG-Leitungen. Im Wesentlichen unterhalb des Trägers, also auf der der Haut zugewandten Seite, ist ein Unterknopf (Eyelet) der der Übernahme elektrischer Potenziale direkt vom Gel (Kontaktmedium) oder zur Übergabe an das Gel dient. Dabei ist das Eyelet sowohl elektrisch wie mechanisch mit dem Stud verbunden und zwar im Allgemeinen durch Vernieten der beiden Teile, derart, dass zwischen einem flanschartig seitlich abstehenden Haltebereich des Studs und einem ebensolchen Haltebereich des Eyelets das Trägermaterial der Elektrode fest eingeklemmt ist. Eine solche Konstruktion bietet einerseits einen guten mechanischen Halt des Anschlusselementes am Träger der Elektrode und erlaubt es andererseits, das Eyelet aus Materialien zu fertigen, die für eine Signalelektrode elektrische günstige Eigenschaften aufweisen, beispielsweise kann es dazu mit Silber beschichtet sein, wobei die Silberschicht wiederum ganzflächig oder zumindest in einem Teilbereich, der mit dem Gel in Kontakt steht, mit einer Schicht aus Silber / Silberchlorid (Ag /AgCl) überdeckt ist. Es besteht auch die Möglichkeit, dass das Eyelet das Gel nicht direkt berührt. Dann ist bei diesen sogenannten Off-Center-Signalelektroden ein Querleiter vorgesehen, der das Eyelet mit dem Gel verbindet.

Eine solche Off-Center-Signalelektrode ist beispielsweise aus der WO 2006/053366 A1 bekannt.

Die US 4,777,954 A offenbart eine medizinische Elektrode mit einem zweiteiligen Anschlusselement.

In der DE 20 2007 006 112 U1 ist eine Elektrode gezeigt, bei welcher das Anschlusselement an der Signalleitung angeordnet ist. An der Elektrode befindet sich eine Öffnung, in die das an der Signalleitung befindliche Anschlusselement eingebracht wird.

Die WO 01/17423 A2 zeigt eine Elektrode mit einem einteiligen Anschlusselement. Das Anschlusselement wird dabei mittels eines Flanschs befestigt, ein Kontaktmedium zum Kontaktieren der Haut wird direkt auf das Anschlusselement aufgebracht.

Die WO 2016/001393 A1 offenbart wiederum eine Elektrode mit zweiteiligem Anschlusselement.

US 4 117 846 A offenbart eine Elektrode mit seitlichem Versatz und einem Querleiter, der direkt mit dem Anschlusselement galvanisch verbunden ist.

Bei solche Elektroden nach dem Stand der Technik ist es oft der Fall, dass die Kontaktstelle zur Haut hinsichtlich verschiedener Faktoren nicht immer die gewünschten Erfordernisse erfüllt. Außerdem sind Elektroden nach dem Stand der Technik teuer - wobei bei derartigen Massenartikeln bereits geringfügige Preisunterschiede ins Gewicht fallen.

Aufgabe der Erfindung ist es daher, eine Elektrode des eingangs genannten Typs zu schaffen, die sich kostengünstiger herstellen lässt und die trotz der kostengünstigen Herstellung eine gute mechanische Verankerung des Anschlusselements in der Elektrode sowie gute elektrische Eigenschaften bereitstellt.

Erfindungsgemäß wird dies durch eine Elektrode gemäß Anspruch 1 erreicht.

Im Gegensatz zur bisher häufig üblichen zweiteiligen Konstruktion, bei der das Anschlusselement aus zwei miteinander vernieteten Teilen (Stud und Eyelet) besteht, ist erfindungsgemäß nunmehr ein einziger Teil als Anschlusselement vorgesehen, der einerseits die Anschlussstelle zum lösbaren Anschließen eines Signalleiters bereitstellt und andererseits mit dem elektrischen Querleiter (vorzugsweise galvanisch) in Verbindung steht. Dabei kann der einzige Teil des Anschlusselementes durchaus selbst aus mehreren Materialien bestehen, zum Beispiel aus vernickeltem Messing oder einem mit leitfähigen Material (insbesondere Kohlenstofffasern) dotiertem Kunststoff. Er stellt aber im Gegensatz zum bisherigen zweiteiligen Aufbau aus Eyelet und Stud eine bauliche Einheit im Sinne eines einzigen Teiles da.

Besonders bevorzugt ist eine Ausbildung des Anschlusselementes, bei dem dieses derart ausgebildet ist, dass das Anschlusselement einen im Wesentlichen kugelförmigen Kopf, einen daran anschließenden, im Durchmesser reduzierten Hals und am dem Kopf abgewandten Ende des Halses einen flanschförmig seitlich abstehenden Haltebereich aufweist. Über den kugelkopfförmigen Kopf lassen sich Standardsignalleiter leicht lösbar anschließen. Der im Durchmesser reduzierte Hals wird (vorzugsweise ohne seitliche Berührung) durch eine Öffnung im Träger geführt, während der flanschförmig seitlich abstehende Haltebereich mit der Unterseite des Trägers beziehungsweise einem darauf angebrachten schichtförmigen Querleiter verbunden, vorzugsweise verklebt ist. Der im Durchmesser erweiterte flanschförmig seitlich abstehende Haltebereich hält das Anschlusselement auch durch hohe Zugbelastungen sicher am Trägermaterial fest.

Um auch bei Druckbelastungen auf das Anschlusselement einen guten Halt zu gewährleisten, ist vorzugsweise vorgesehen, dass das Anschlusselement einen seitlich abstehenden Haltebereich aufweist, der zwischen einer Stützlage und dem Träger angeordnet ist, wobei sich die Stützlage seitlich über den Haltebereich des Anschlusselementes hinaus erstreckt und dort fest mit dem Träger verbunden - vorzugsweise verklebt - ist.

Durch Druckbelastungen auf das Anschlusselement ausgeübte Kräfte werden einerseits von der Verklebung des Haltebereichs mit der Unterseite des Trägers oder dem dort vorgesehenen Querleiter abgefangen, andererseits von der Stützlage, die diese Kräfte seitlich in den Träger ableitet.

An die elektrischen Eigenschaften des Halteelementes sind beim Erfindungsgegenstand keine hohen Anforderungen gestellt. Er kann daher aus kostengünstigem Material, beispielsweise aus einem einfachen Metallblech bestehen. Das Halteelement braucht nämlich keine besonderen elektrischen Eigenschaften aufweisen, denn diese für Bioelektroden günstigen elektrischen Eigenschaften kann lediglich der Querleiter aufweisen, der mit dem elektrischen Kontaktmedium in Verbindung steht.

Um bei einer Elektrode ein geringes Rauschen und eine Depolarisation im Falle einer Defibrillation zu erreichen, werden aktuell Redoxpaare verwendet. Diese können oxidiert bzw. reduziert werden und nehmen dabei mindestens ein Elektron auf oder geben mindestens ein Elektron ab. Aktuell werden verschiedenste Substanzen für diese Depolarisation verwendet. Am häufigsten werden Silber / Silberchlorid und Zinn / Zinnchlorid verwendet. Für die vorliegende Erfindung sind allerdings sämtliche Redoxpaare denkbar, welche eine Depolarisation der Elektrode ermöglichen. Dabei können die Redoxpaare aktiv beigemengt oder durch Reaktionen möglicherweise in situ erzeugt werden.

Nachdem beispielsweise Silber / Silberchlorid eine relativ teure Substanz ist, reicht es aus, wenn gemäß der Erfindung vorgesehen ist, dass der Querleiter zumindest zwei unterschiedliche elektrisch leitende Materialien aufweist, von denen eines mit dem Anschlusselement galvanisch verbunden ist und von denen ein anderes mit dem Kontaktmedium galvanisch verbunden ist.

Durch die Maßnahme den Querleiter aus zumindest zwei unterschiedlichen Materialien aufzubauen, kann man weiter Kosten sparen. Die die eigentliche Querleitung kann man nämlich relativ kostengünstige Materialien, wie beispielsweise Metall oder mit leitenden Karbonfasern versehenen Kunststoff verwenden, während man an dem für die günstigen elektrischen Eigenschaften der Bioelektrode kritischen Übergangsbereich zum elektrischen Kontaktmedium (insbesondere Gel) ein zweites Material wie beispielsweise Silber / Silberchlorid verwenden kann. Es reicht aus, wenn dieses Material nur lokal in diesem Bereich vorhanden ist.

Insgesamt liegt der Erfindung der Grundgedanke zu Grunde, das Anschlusselement für den Signalleiter so auszubilden, dass es gut in der Elektrode verankert ist, während es auf die elektrischen Eigenschaften weniger ankommt und damit kostengünstige Materialien verwendet werden können. Andererseits können die für die günstige elektrische Signalleitung vorgesehenen teureren Materialen lediglich in dem elektrisch kritischen Bereich am Übergang zum elektrischen Kontaktmedium (Gel) verwendet werden. Diese Aufgabe übernimmt der Querleiter. Ganz kurz formuliert würde man sagen, das elektrisch leitende Anschlusselement ist, abgesehen von der Grundeigenschaft der elektrischen Leitung hauptsächlich für die "Mechanik" verantwortlich. Beim Querleiter ist es umgekehrt: er braucht keine besondere mechanischen Eigenschaften zu erfüllen und lediglich im Bereich der Übergangsstelle zum elektrischen Kontaktmedium (Gel) aus dafür günstigen Materialien bestehen. Insofern ist der Querleiter ohne besondere mechanische Aufgaben für die "Elektrik" zuständig.

Weitere Vorteile und Einzelheiten der Erfindung werden anhand der nachfolgenden Figurenbeschreibung näher erläutert:
Die Figur 1 zeigt in einer schematischen Unteransicht (später die der Haut zugewandte Seite) die Herstellungsschritte eines Ausführungsbeispiels einer erfindungsgemäßen Elektrode bis zur fertigen Elektrode.
Die Figur 2 zeigt eine entsprechende Draufsicht, wobei nur ein Teil der Prozessschritte in einer Draufsicht dargestellt ist.
Die Figur 3 zeigt die Abfolge der Schnitte gemäß der Linie AA der Figur 1, wobei die Darstellung zur besseren Visualisierung als schematische Darstellung zu verstehen ist. In der Realität kann die Schichtenfolge eine andere Dimensionierung aufweisen, wie sie bei medizinischen Elektroden üblich ist.
Die Figuren 4, 5 und 6 zeigen im Wesentlichen dieselben Darstellungen wie in den Figuren 1, 2 und 3, jedoch für ein anderes Ausführungsbeispiel.
Die Figuren 7 und 8 zeigen die Unteransicht auf Ausführungsbeispiele eines elektrischen Anschlusselementes.

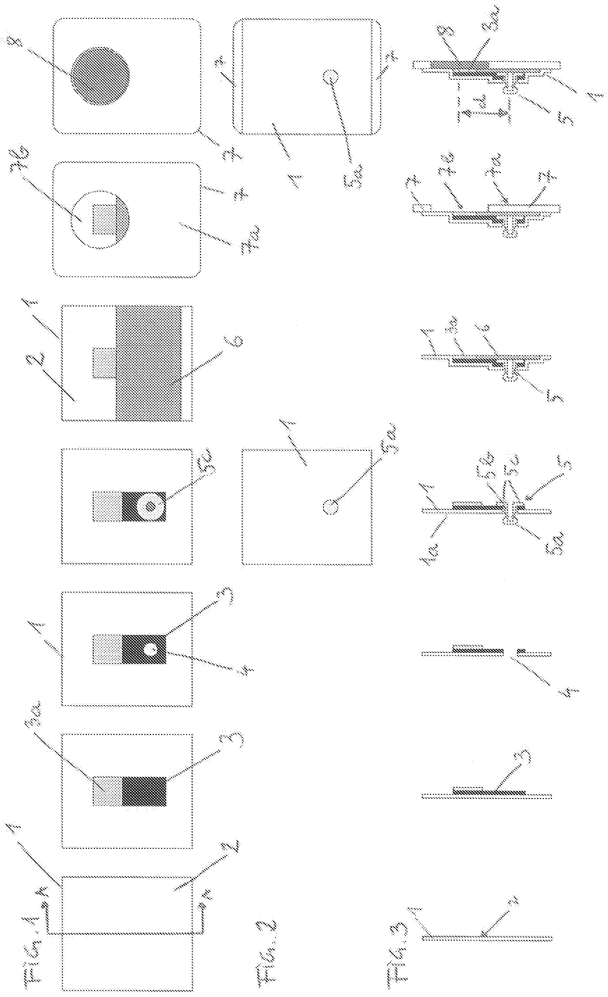

Unter Bezugnahme auf die Figuren 1 bis 3 wird nun der Verfahrensablauf zur Herstellung eines Ausführungsbeispiels einer erfindungsgemäßen Elektrode zum Anbringen auf der menschlichen Haut näher erläutert.

Ausgegangen wird von einem elektrisch nichtleitenden Träger 1. Das Trägermaterial dient zur Verankerung der elektrischen Komponenten der Elektrode. Es kann beispielsweise aus einer (flexiblen) Folie (beispielsweise aus PET oder TPU) bestehen, die auf der in der Zeichnung der Figur 1 nach oben weisenden Unterseite mit einem Klebstoff 2 beschichtet ist, der beispielsweise selbstklebend (pressure sensitive adhesive) oder thermoaktivierbar (hot melt) ausgeführt sein kann.

Auf dieses Trägermaterial wird nun in einem nächsten Schritt ein streifenförmiger Querleiter 3 befestigt, insbesondere verklebt. Der Querleiter weist gemäß einer bevorzugten Variante der Erfindung zwei unterschiedlich elektrisch leitende Materialien auf, von denen eines später mit dem elektrischen Anschlusselement galvanisch verbunden wird und von denen das andere mit dem Kontaktmedium (Gel) galvanisch verbunden wird.

Bei dem dargestellten Ausführungsbeispiel handelt es sich um einen schwarz dargestellten, streifenförmigen Leiter aus einem mit leitfähigen Karbonfasern dotiertem Kunststoff. Im Bereich der späteren Kontaktstelle mit dem elektrischen Kontaktmedium (Gel) ist dieser Querleiter 3 (erstes Material mit einem zweiten elektrisch leitenden Material) beispielsweise einer Schicht 3a aus Silber / Silberchlorid oder Zinn / Zinnchlorid oder einem anderen Redoxpaar beschichtet.

Diese für die günstigen elektrischen Eigenschaften von der Kontaktstelle zum späteren Gel vorgesehene Schicht 3a ist nur dort vorgesehen. wo später das Gel vorgesehen ist. Ansonsten reicht ein "normaler" Leiter 3, der wesentlich kostengünstiger ist, aus, um die elektrische Verbindung mit dem im Folgenden beschriebenen elektrischen Anschlusselement herzustellen.

In einem weiteren Schritt wird nun eine Bohrung 4 durch den elektrischen Querleiter 3 und den Träger 1 vorgesehen. Das kann beispielsweise durch Ausstanzen geschehen. Anschließend folgt die Einbringung des elektrisch leitenden Anschlusselementes 5, das eine im Wesentlichen kugelkopfförmige Anschlussstelle 5a zum lösbaren Anschließen eines nicht dargestellten handelsüblichen Signalleiters aufweist und das über die Oberseite 1a des Trägers 1 vorsteht.

Das elektrische Anschlusselement weist beim dargestellten Ausführungsbeispiel im Anschluss an den im Wesentlichen kugelförmigen Kopf 5a einen im Durchmesser reduzierten Hals 5b auf, an dem sich letztlich an dem Kopf 5a abgewandten Ende ein flanschförmig seitlich abstehender Haltebereich 5c befindet.

Insgesamt ist der seitlich abstehende flanschförmige Haltebereich 5c im Wesentlichen tellerförmig ausgebildet. Er dient einerseits zur elektrischen Kontaktierung mit dem Querleiter 3, in dem dieser zwischen Träger 1 und tellerförmigen Flansch 5c "eingeklemmt" wird. Andererseits dient der tellerförmige Flansch zum Herstellen des mechanischen Halts des elektrischen Anschlusselementes insbesondere gegen Zugbelastungen, die durch ein Signalkabel auf den Kopf 5a und damit das gesamte Anschlusselement 5 ausgeübt werden können.

Bevorzugt ist vorgesehen, dass zwischen dem Querleiter 3 und dem Anschlusselement 5 bzw. einem davon seitlich abstehenden Flansch 5c ein elektrisch leitender Kleber vorgesehen ist.

Im Gegensatz zu den bisher üblichen vernieteten zweiteiligen Anschlusselementen bestehend aus oben vorstehendem Stud (Oberknopf) und dem untenliegenden Eyelet (Unterknopf) kommt erfindungsgemäß ein Anschlusselement 5 zum Einsatz, das aus einem einzigen Teil besteht, das einerseits mit dem elektrischen Querleiter 3 in Verbindung stehen und andererseits die Anschlussstelle 5a zum lösbaren Anschließen eines (hier nicht dargestellten) Signalleiters aufweist. Damit ist eine kostengünstige Herstellung der Elektrode möglich, weil das meist kostenintensive Eyelet (Unterknopf) entfallen kann. Für die mechanische Verankerung reicht die einteilige Ausbildung des Anschlusselementes aus.

Die Anforderungen an die elektrischen Eigenschaften sind gering. Damit können einfache Konstruktionen, wie beispielsweise ein tiefgezogenes Metallteil als Anschlusselement 5 verwendet werden. Die etwas diffizileren elektrischen Aufgaben übernimmt also hier nicht das sonst übliche Eyelet (Unterknopf) sondern jenes Ende des Querleiters 3, das mit dem später aufgebrachten elektrischen Kontaktmedium (Gel) in Verbindung steht. Es erfolgt also eine Trennung der Aufgaben. Das elektrische Anschlusselement ist abgesehen von der Basiseigenschaft elektrisch leitend zu sein, im Wesentlichen für den mechanischen Halt in der Elektrode verantwortlich, während der Querleiter von mechanischen Aufgaben weitgehend befreit ist. Das erlaubt es eine günstige Materialwahl zu treffen. Insbesondere ist es möglich, teurere - aus elektrischer Sicht günstige - Materialien nur dort (Stelle 3a) vorzusehen, wo später der Kontakt mit dem Gel erfolgt.

Das elektrisch leitende Anschlusselement kann - wie bereits erwähnt - aus einem tiefgezogenen Metallblech bestehen. Es ist dann im Inneren zumindest teilweise hohl. Es kann aber auch aus einem leitfähigen Kunststoff bestehen, beispielsweise aus ABS, das mit leitfähigen Karbonfasern dotiert ist.

Günstigerweise wird man das Anschlusselement im Wesentlichen rotationssymmetrisch ausbilden. Andere Varianten sind auch möglich.

Um das elektrische Anschlusselement 5c endgültig in der Elektrode zu fixieren und insbesondere auch gegen Druckbelastungen auf den Kopf 5a zu sichern, wird in einem nächsten Schritt eine Stützlage 6 aufgebracht. Diese Stützlage 6 kann beispielsweise aus einem Doppelklebeband bestehen, das auf der in der Figur 1 untenliegenden Seite mit dem Anschlusselement 5 (konkret mit dem tellerförmigen Haltebereich 5c) und mit Bereichen der Unterseite 1a des Trägers 1 verklebt ist.

Dabei kann Druck auf die Schichten ausgeübt werden, sodass sich diese entsprechend konturieren und miteinander verbinden. Der in Figur 3 gezeigte Querschnitt nach Anbringen der Stützlage 6 mit den dort gezeigten Kanten ist allerdings lediglich als schematische Darstellung zu sehen. In Wirklichkeit sind die Schichtdicken meist geringer und die Verläufe der Schichten sind wesentlich abgerundeter.

Auf das Doppelklebeband 6, welches mit dem Träger 1, dem elektrischen Querleiter 3 und dem Haltebereich 5c des elektrischen Anschlusselements 5 auf der einen Seite verklebt ist, wird nun auf der anderen Seite mit einer Pflasterschicht 7 verklebt, wobei die Pflasterschicht vorzugsweise mittels einer patientenseitigen Beschichtung aus biokompatiblem Klebstoff auf die Haut aufklebbar ist, um die Elektrode zu fixieren.

Entgegen den dargestellten Ausführungsbeispielen kann die Stützlage auch direkt von der Pflasterschicht gebildet werden (ohne zwischengelegtes Doppelklebeband). Dabei ist es auch möglich, die Pflasterschicht über eine auf ihr aufgebrachte Schicht aus Selbstkleber oder aus einem thermoaktivierbaren Kleber mit dem Träger 1 und dem Haltebereich 5c des Anschlusselementes 5 zu verkleben.

Nun zurück zum Ausführungsbeispiel gemäß den Figuren 1 bis 3. Das dort dargestellte Pflastermaterial 7 wird nicht nur über das Doppelklebeband 6 sondern auch über den Kleber auf der Unterseite 2 des Trägers 1 mit diesem fest verbunden.

Das Pflastermaterial dient letztlich dazu, die Elektrode auf der Patientenhaut zu fixieren. Geeignete Pflastermaterialien können beispielsweise aus einer Folie (beispielsweise PE), aus einem Schaumband (beispielsweise PE-Schaum) oder aus Vliesstoffen bestehen. Die Pflastermaterialien sind üblicherweise patientenseitig mit einem biokompatiblen Klebstoff 7a beschichten.

In einem letzten Herstellungsschritt der Elektrode gemäß den Figuren 1 bis 3 erfolgt die Einbringung des elektrischen Kontaktmediums in eine dafür vorgesehene Aussparung 7b im Pflastermaterial 7. Das elektrische Kontaktmedium ermöglicht die (vorzugsweise ionen-basierte) Leitung von körpergenerierten elektrischen Potenzialen oder von gerätegenerierten Mess- oder Stimulationsströmen von der Körperoberfläche (Haut) zum elektrischen Kontaktelement und umgekehrt. Das Kontaktmedium kann beispielsweise aus einem mit Chloriden dotiertem Gel bestehen, das entweder in mehr oder weniger flüssiger Form (mehr oder weniger geliert) oder als quervernetzte Polymer-Matrix (Hydrogel) vorliegt. Es ist aber auch möglich, das elektrische Kontaktmedium mit anderen Mitteln zu erzeugen, beispielsweise als leitfähiger Kleber oder als mit Salzlösung gefüllter Schwamm.

Jedenfalls wird das elektrische Kontaktmedium 8, wie es der letzte Schritt in den Figuren 1 bis 3 zeigt, in die Aussparung 7b eingebracht. Es kontaktiert darin den Endbereich 3a (dortiges zweites Material des Querleiters, insbesondere Silber / Silberchlorid).

Das Zusammenwirken des speziell ausgebildeten Endbereichs des Querleiters 3, insbesondere die Beschichtung mit Silber / Silberchlorid oder einem anderen geeigneten Material einerseits und dem Material des elektrisch leitenden Kontaktmediums 8 andererseits, erlaubt es günstige elektrische Eigenschaften der Elektrode, wie beispielsweise rauschfreie Signalübertragung oder depolarisierende Wirkungen zu erzielen, wobei der Einsatz des relativ teuren zweiten Materials 3a am Ende des Querleiters 3 auf jenen Bereich beschränkt bleiben kann, in den ein Kontakt mit dem Kontaktmedium 8 erfolgt. Das senkt die Kosten weiter.

Insgesamt ergibt sich bei der Herstellung gemäß den Figuren 1 bis 3 eine "dezentrale" Elektrode, bei der das Anschlusselement 5 einerseits und das Kontaktmedium 8 (Gel) andererseits an seitlich gegeneinander versetzten Stellen (Abstand d) am Träger 1 angeordnet sind.

Die für das Ausführungsbeispiel gemäß den Figuren 1 bis 3 wesentlichen Verfahrensschritt e sind die folgenden:
- Anbringen, vorzugsweise thermoaktiviertes Verkleben, eines streifenförmigen Querleiters auf der der Haut zugewandten Unterseite eines elektrisch nichtleitenden Trägers,
- Herstellen, vorzugsweise Ausstanzen einer durchgehenden Öffnung durch den Querleiter und den Träger,
- Einbringen eines einteiligen Anschlusselementes von der Unterseite des Träger aus in die Öffnung, derart dass auf der gegenüberliegenden Oberseite des Trägers eine Anschlussstelle für einen Signalleiter vorsteht und das Anschlusselement mit einem seitlich abstehenden - vorzugsweise tellerförmigen - Haltebereich am Querleiter anliegt,
- Überdecken des Haltebereichs des Anschlusselements mit einer Stützlage, die seitlich neben dem Haltebereich mit dem Träger verklebt wird.

Schließlich werden dann zur Fertigstellung der Elektrode noch folgende Schritte gesetzt:
- Anbringen - vorzugsweise Verkleben - einer hautseitig klebenden Pflasterschicht mit dem Träger und/oder der Stützlage,
- Einbringen von einem elektrischen Kontaktmedium - vorzugsweise einem Gel - in eine Aussparung der Pflasterschicht, derart dass der darunterliegende Querleiter kontaktiert wird.

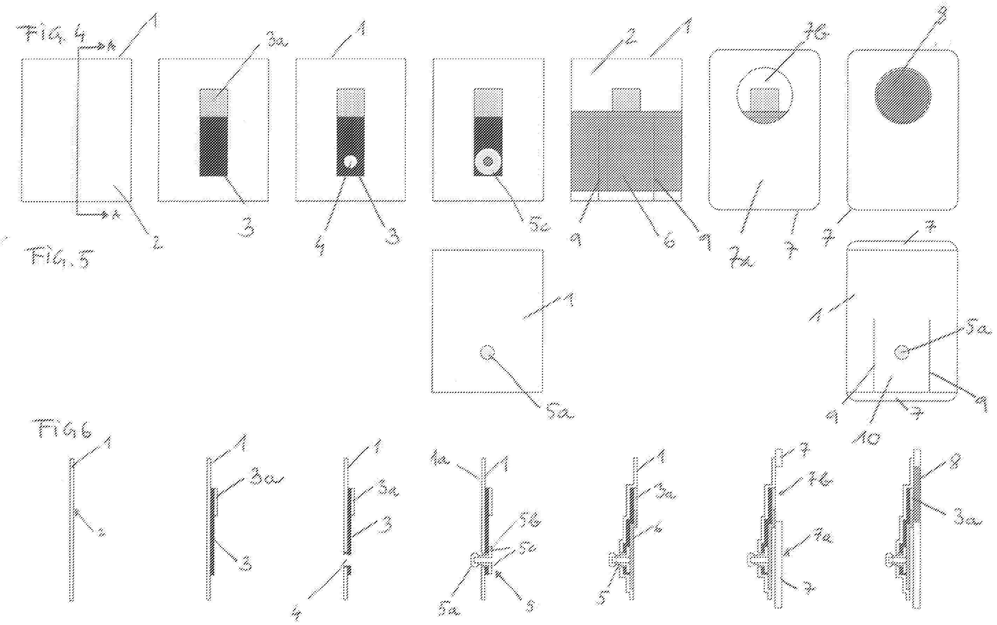

Bei dem in den Figuren 4 bis 6 dargestellten Ausführungsbeispiel stimmen die meisten Verfahrensschritte mit denen in Figur 1 bis 3 überein, weshalb auch gleiche Bezugszeichen gleiche Teile bezeichnen.

Der Unterschied besteht im Wesentlichen im Schritt 5. Es werden hier nämlich gemäß der Figur 4 zwei Einschnitte 9 durch den gesamten Verbund vorgenommen. Im nächsten Schritt 6 wird dann das Pflastermaterial 7 nur im oberen Bereich und unten an den "Flügeln" festgeklebt (beispielsweise durch lokale thermische Aktivierung), nicht jedoch im Bereich der dadurch beweglich bleibenden Lasche.

Insgesamt ergibt sich beim Ausführungsbeispiel gemäß den Figuren 4 bis 6 eine bewegliche Lasche 10, die das Anschlusselement 5 mit der Anschlussstelle 5a trägt. Diese bewegliche Lasche kann Zugbelastungen auf den nichtdargestellten Signalleiter und damit auf die Anschlussstelle 5a ausgleichen, sodass sich diese nicht voll auf die Elektrode überträgt. Insgesamt wird damit die Haftung der Elektrode auf der Haut des Patienten verbessert.

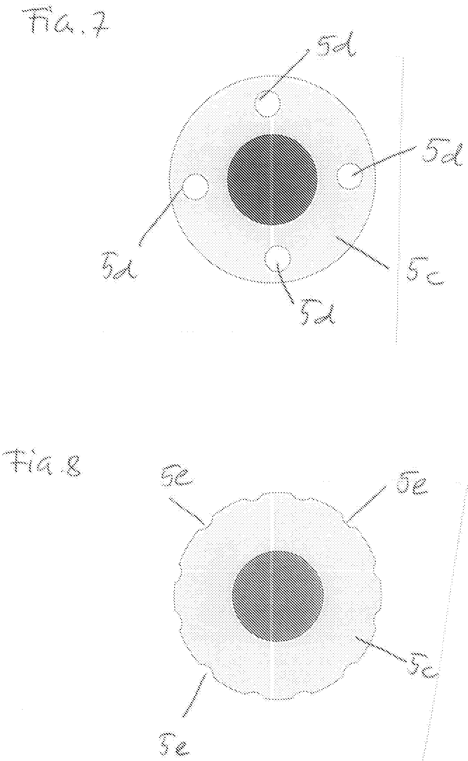

In Figur 7 ist ein Ausführungsbeispiel eines Haltebereichs 5c gezeigt, der flanschförmig seitlich vom Anschlusselement 5 absteht. Dieser Haltebereich bzw. Flansch weist Bohrungen 5d auf. Beim Verkleben der Elektrode dringt Kleber in diese Bohrungen ein und verbessert damit die Haftung und Verdrehsicherheit des Anschlusselementes mit den Teilen der übrigen Elektrode.

Zum gleichen Zweck dient die in Figur 8 dargestellte Ausführungsform. Hier sind am Umfangsrand des tellerförmig ausgebildeten Haltebereichs Einbuchtungen 5e vorgesehen. Auch in diese dringt der Hot Melt-Kleber ein und verbessert damit die Haftung.

Der nicht gezeigte Signalleiter ist in bekannter Weise ausgebildet und besteht üblicherweise aus einem isolierten, flexiblen Kabel, das von einer Auswerteinrichtung oder Stromzufuhreinrichtung zur Elektrode führt. Der Signalleiter selbst ist nicht Teil der Elektrode, also gesondert von dieser und ihrem Anschlusselement ausgebildet. An seinem elektrodenseitigen Ende trägt der Signalleiter meist ein Kupplungsstück, über das er mechanisch und elektrisch lösbar mit der Anschlussstelle des Anschlusselementes der Elektrode verbindbar ist, das seinerseits bevorzugt an der Elektrode vormontiert und dauerhaft mit dieser verbunden ist.

## Patentansprüche

1. Elektrode zum Anbringen auf der menschlichen Haut mit einem elektrisch nichtleitenden Träger, der auf seiner der Haut abgewandter Oberseite ein vorstehendes, elektrisch leitendes Anschlusselement mit einer Anschlussstelle zum lösbaren Anschließen eines Signalleiters aufweist, wobei ein zumindest teilweise auf der gegenüberliegenden Unterseite des Trägers verlaufender Querleiter vorgesehen ist, der elektrisch mit dem Anschlusselement sowie mit einem der Haut zugewandten Kontaktmedium in Verbindung steht, wobei das Anschlusselement (5) aus einem einzigen Teil besteht, das einerseits mit dem elektrischen Querleiter (3) in Verbindung steht und andererseits die Anschlussstelle (5a) zum lösbaren Anschließen eines gesonderten Signalleiters aufweist, wobei das Anschlusselement (5) einerseits und das Kontaktmedium (8) andererseits an seitlich gegeneinander versetzten Stellen (Abstand d) am Träger (1) angeordnet sind, wobei das Anschlusselement (5)
- vorzugsweise mit einem im Durchmesser verjüngten Hals (5b) - durch eine Öffnung (4) im Träger (1) hindurch ragt, und dass das Anschlusselement (5) - abgesehen von einer allfälligen seitlichen Anlage im Bereich der Öffnung - lediglich auf der der Haut zugewandten Unterseite des Trägers (1) mit diesem unter Zwischenschaltung eines flächig ausgebildeten Querleiters (3) verbunden ist, **gekennzeichnet dadurch, dass** der Querleiter (3) zumindest zwei unterschiedliche elektrisch leitende Materialien aufweist, von denen eines (3) mit dem Anschlusselement (5) galvanisch verbunden ist und von denen ein anderes (3a) mit dem Kontaktmedium (8) galvanisch verbunden ist.

2. Elektrode nach Anspruch 1, **dadurch gekennzeichnet, dass** das Anschlusselement (5) aus Metall, vorzugsweise einem tiefgezogenen Metallblech, oder aus leitfähigem Kunststoff, vorzugsweise mit leitfähigen Karbonfasern dotiertem ABS, besteht.

3. Elektrode nach Anspruch 1 oder 2, **dadurch gekennzeichnet, dass** das Anschlusselement (5) einen im Wesentlichen kugelförmigen Kopf (5a), einen daran anschließenden, im Durchmesser reduzierten Hals (5b) und am dem Kopf (5a) abgewandten Ende des Halses (5b) einen flanschförmig seitlich abstehenden Haltebereich (5c) aufweist.

4. Elektrode nach einem der Ansprüche 1 bis 3, **dadurch gekennzeichnet, dass** das - vorzugsweise in einer Aussparung (7b) einer Pflasterschicht (7) angeordnete - Kontaktmedium (8) ein - vorzugsweise mit Chloriden dotiertes Gel ist, als leitfähiger Kleber ausgebildet ist oder als ein mit Salzlösung gefüllter Schwamm ausgebildet ist.

5. Elektrode nach einem der Ansprüche 1 bis 4, **dadurch gekennzeichnet, dass** das Anschlusselement (5) einen seitlich abstehenden Haltebereich (5c) aufweist, der zwischen einer Stützlage (6) und dem Träger (1) angeordnet ist, wobei sich die Stützlage (6) seitlich über den Haltebereich (5c) des Anschlusselementes (5) hinaus erstreckt und dort fest mit dem Träger (1) verbunden - vorzugsweise verklebt - ist.

6. Elektrode nach Anspruch 5, **dadurch gekennzeichnet, dass** die Stützlage (6) als Doppelklebeband oder als Band aus einem thermoaktivierbaren Klebstoff oder als Band aus einem thermoplastischen, zur direkten thermoplastischen Verbindung mit dem Träger geeigneten Material ausgeführt ist, das auf der einen Seite mit dem Anschlusselement (5) und dem Träger (1) verklebt ist.

7. Elektrode nach Anspruch 5, **dadurch gekennzeichnet, dass** die Stützlage (6) von einer Pflasterschicht (7) gebildet ist, wobei die Pflasterschicht - vorzugsweise mittels einer patientenseitigen Beschichtung aus biokompatiblem Klebstoff (7a) - auf die Haut aufklebbar ist, um die Elektrode zu fixieren.

8. Elektrode nach einem der Ansprüche 1 bis 7, **dadurch gekennzeichnet, dass** der Querleiter (3) als vorzugsweise streifenförmige Schicht aus einem ersten elektrisch leitenden Material ausgebildet ist, die im Bereich des Kontaktmediums (8) - und vorzugsweise nur dort - mit einem zweiten elektrisch leitenden Material (3a) versehen, vorzugsweise beschichtet ist.

9. Elektrode nach Anspruch 8, **dadurch gekennzeichnet, dass** das erste elektrisch leitende Material (3) ein Metall oder eine Metalllegierung, eine beispielsweise durch leitfähige Karbonfasern durchgängig oder oberflächlich leitende Kunstfolie, oder ein durchgängig oder oberflächlich leitendes textiles Material ist.

10. Elektrode nach Anspruch 8 oder 9, **dadurch gekennzeichnet, dass** das zweite Material (3a) von einem Paar Silber / Silberchlorid oder Zinn / Zinnchlorid oder einen anderen zur Depolarisation der Elektrode geeigneten Redoxpaar augebildet ist.

11. Elektrode nach einem der Ansprüche 1 bis 10, **dadurch gekennzeichnet, dass** Träger (1) samt einer allfällig damit verbundenen Stützlage (6) im Bereich neben dem Anschlusselement (5) zumindest einen Einschnitt (9) aufweist, der eine Beweglichkeit des Anschlusselementes (5) gegenüber einer zum Verkleben mit der Haut vorgesehenen Pflasterschicht (7) erlaubt.

12. Elektrode nach einem der Ansprüche 1 bis 11, **dadurch gekennzeichnet, dass** das Anschlusselement (5) an der Elektrode vormontiert und dauerhaft mit dieser verbunden ist.

13. Verfahren zum Herstellen einer Elektrode zum Anbringen auf der menschlichen Haut nach einem der Ansprüche 1 bis 12, umfassend folgende Schritte
- Anbringen, vorzugsweise thermoaktiviertes Verkleben, eines streifenförmigen Querleiters auf der der Haut zugewandten Unterseite eines elektrisch nichtleitenden Trägers,
- Herstellen, vorzugsweise Ausstanzen einer durchgehenden Öffnung durch den Querleiter und den Träger,
- Einbringen eines einteiligen Anschlusselementes von der Unterseite des Träger aus in die Öffnung, derart dass auf der gegenüberliegenden Oberseite des Trägers eine Anschlussstelle für einen Signalleiter vorsteht und das Anschlusselement mit einem seitlich abstehenden - vorzugsweise tellerförmigen - Haltebereich am Querleiter anliegt,
- Überdecken des Haltebereichs des Anschlusselements mit einer Stützlage, die seitlich neben dem Haltebereich mit dem Träger verklebt wird.

14. Verfahren nach Anspruch 13, **gekennzeichnet durch** folgende weitere Schritte:
- Anbringen - vorzugsweise Verkleben - einer hautseitig klebenden Pflasterschicht mit dem Träger und/oder der Stützlage,
- Einbringen von einem elektrischen Kontaktmedium - vorzugsweise einem Gel - in eine Aussparung der Pflasterschicht, derart dass der darunterliegende Querleiter kontaktiert wird.

## Claims

1. An electrode for application to human skin, comprising an electrically non-conductive carrier, which has an electrically conductive connecting element projecting from the top side of the carrier remote from the skin, the connecting element having a connection location for releasably connecting a separate signal conductor, wherein a transverse conductor extending at least partially on the underside of the carrier, and being arranged so as to electrically connect the connecting element to a contact medium facing the skin, wherein the connecting element (5) is formed of a single part which one on hand is in connected relationship with the transverse conductor (3) and on the other hand has the connection location (5a) for releasably connecting the separate signal conductor, wherein the connecting element (5) on the one hand and the contact medium (8) on the other hand are arranged at laterally mutually displaced locations (distance d) on the carrier (1), wherein the connecting element (5) projects through an opening (4) in the carrier (1), preferably with a neck (5b) that tapers in diameter, and that the connecting element (5), apart from any lateral contact in the area of the opening, is connected to the carrier (1) only on the underside of the carrier facing the skin, with a flat transverse conductor (3) interposed between them, **characterized in that** the transverse conductor (3) comprises at least two different electrically conductive materials, one of which (3) is galvanically connected to the connecting element (5) and another of which (3a) is galvanically connected to the contact medium (8).

2. The electrode according to claim 1, **characterized in that** the connecting element (5) comprises a metal, preferably a deep-drawn metal sheet, or conductive plastic, preferably with ABS doped with conductive carbon fibers.

3. The electrode according to claim 1 or 2, **characterized in that** the connecting element (5) has a substantially spherical head (5a), a reduced-diameter neck (5b) adjoining the head, and a laterally projecting flange-shaped holding region (5c) that is arranged at an end of the neck (5b) remote from the head (5a).

4. The electrode according to one of claims 1 to 3, **characterized in that** the contact medium (8), preferably arranged in a recess (7b) of a plaster layer (7), is a gel, preferably doped with chlorides, is designed as a conductive adhesive or a saline-filled sponge.

5. The electrode according to one of claims 1 to 4, **characterized in that** the connecting element (5) has a laterally protruding holding area (5c) which is arranged between a support layer (6) and the carrier (1), wherein the support layer (6) extends laterally beyond holding area (5c) of the connecting element (5) and is firmly connected there to the carrier (1), preferably by being glued.

6. The electrode according to claim 5, **characterized in that** the support layer (6) is designed as double-sided adhesive tape or as tape made of a thermally activatable adhesive or as tape made of a thermoplastic material suitable for direct thermoplastic connection to the carrier, which is glued on one side to the connecting element (5) and the carrier (1).

7. The electrode according to claim 5, **characterized in that** the support layer (6) is formed by a plaster layer (7), wherein the player layer can be glued to the skin, preferably by means of a patient-side coating of biocompatible adhesive (7a), in order to fix the electrode in place.

8. The electrode according to one of claims 1 to 7, **characterized in that** the transverse conductor (3) is designed as a preferably strip-shaped layer made of a first electrically conductive material, which is provided with, preferably coated with, a second electrically conductive material (3a) in the area of the contact medium (8) and preferably only there.

9. The electrode according to claim 8, **characterized in that** the first electrically conductive material (3) is a metal or a metal alloy, a plastic film which is conductive throughout or superficially, for example through conductive carbon fibers, or a textile material which is conductive throughout or superficially.

10. The electrode according to claim 8 or 9, **characterized in that** the second material (3a) is formed by a pair of silver/silver chloride or tin/tin chloride or another redox pair suitable for depolarizing the electrode.

11. The electrode according to one of claims 1 to 10, **characterized in that** the carrier (1) and the support layer (6) connected thereto has at least one incision (9) in a region beside the connecting element, which incision allows mobility of the connecting element (5) with respect to a plaster layer (7) provided for gluing to the skin.

12. The electrode according to one of claims 1 to 11, **characterized in that** the connecting element (5) is pre-mounted on the electrode and permanently connected thereto.

13. A method of producing an electrode for application to human skin according to one of claims 1 to 12, comprising the following steps:
- Applying, preferably thermoactivated bonding, of a strip-shaped transverse conductor to the underside of the electrically non-conductive carrier facing the skin;
- Producing, preferably punching a through opening through the transverse conductor and the carrier;
- Introducing a one-piece connecting element from the underside of the carrier into the through opening, such that the connection location for a signal conductor projects from the opposite top side of the carrier and the connecting element bears with a laterally projecting, preferably plate-shaped, holding region against the transverse conductor; and
- Covering the holding region of the connecting element with a support layer which is glued to the carrier laterally beside the holding region.

14. The method according to claim 13, characterized through the following further steps:
- Applying, preferably by gluing, a plaster layer which is adhesive on the underside of the carrier or the support layer facing the skin; and
- Introducing an electrical contact medium, preferably a gel, into a recess in the plaster layer such that the underlying transverse conductor is contacted.

## Revendications

1. Électrode destinée à être appliquée sur la peau humaine avec un support non conducteur électriquement qui présente sur sa face supérieure opposée à la peau un élément de raccordement électriquement conducteur faisant saillie avec un point de raccordement pour le raccordement amovible d'un conducteur de signal, dans lequel un conducteur transversal s'étendant au moins en partie sur la face inférieure opposée du support est prévu, lequel est électriquement en liaison avec l'élément de raccordement ainsi qu'avec un milieu de contact tourné vers la peau, dans lequel l'élément de raccordement (5) est constitué d'une seule pièce qui est en liaison d'une part avec le conducteur transversal électrique (3) et présente d'autre part le point de raccordement (5a) pour le raccordement amovible d'un conducteur de signal séparé, dans lequel l'élément de raccordement (5) d'une part et le moyen de contact (8) d'autre part sont disposés sur des points décalés latéralement les uns par rapport aux autres (distance d) sur le support (1), dans lequel l'élément de raccordement (5)
- de préférence avec un col (5b) effilé en diamètre - dépasse à travers une ouverture (4) dans le support (1), et que l'élément de raccordement (5) - à l'exception d'un éventuel appui latéral dans la zone de l'ouverture - n'est relié à celui-ci que sur la face inférieure du support (1) tournée vers la peau en intercalant un conducteur transversal (3) plat, **caractérisé en ce que** le conducteur transversal (3) présente au moins deux matériaux électriquement conducteurs différents, dont l'un (3) est relié galvaniquement à l'élément de raccordement (5) et dont un autre (3a) est relié galvaniquement au milieu de contact (8).

2. Électrode selon la revendication 1, **caractérisée en ce que** l'élément de raccordement (5) est constitué de métal, de préférence d'une tôle métallique emboutie, ou de plastique conducteur, de préférence d'ABS dopé de fibres de carbone conductrices.

3. Électrode selon la revendication 1 ou 2, **caractérisée en ce que** l'élément de raccordement (5) présente une tête (5a) sensiblement sphérique, un col (5b) s'y raccordant de diamètre réduit et une zone de maintien (5c) dépassant latéralement en forme de bride sur l'extrémité du col (5b) opposée à la tête (5a).

4. Électrode selon l'une quelconque des revendications 1 à 3, **caractérisée en ce que** le milieu de contact (8) - disposé de préférence dans un évidement (7b) d'une couche de pansement (7) - est un gel dopé de préférence de chlorures, est réalisé en tant que colle conductrice ou est réalisé en tant qu'éponge remplie de solution saline.

5. Électrode selon l'une quelconque des revendications 1 à 4, **caractérisée en ce que** l'élément de raccordement (5) présente une zone de maintien (5c) dépassant latéralement qui est disposée entre une couche d'appui (6) et le support (1), dans laquelle la couche d'appui (6) s'étend latéralement au-delà de la zone de maintien (5c) de l'élément de raccordement (5) et y est reliée de manière solidaire - de préférence est collée - au support (1).

6. Électrode selon la revendication 5, **caractérisée en ce que** la couche d'appui (6) est réalisée sous forme de ruban adhésif double face ou sous forme de ruban composé d'une colle thermoactivable ou sous forme de ruban composé d'un matériau thermoplastique, adapté à une liaison thermoplastique directe avec le support, qui est collé sur un côté à l'élément de raccordement (5) et au support (1).

7. Électrode selon la revendication 5, **caractérisée en ce que** la couche d'appui (6) est formée d'une couche de pansement (7), dans laquelle la couche de pansement peut être collée sur la peau - de préférence au moyen d'un revêtement côté patient composé de colle biocompatible (7a) - pour fixer l'électrode.

8. Électrode selon l'une quelconque des revendications 1 à 7, **caractérisée en ce que** le conducteur transversal (3) est formé en tant que couche de préférence en forme de bande composée d'un premier matériau électriquement conducteur, qui est pourvu, de préférence est revêtu, dans la zone du milieu de contact (8) - et de préférence uniquement là - d'un deuxième matériau électriquement conducteur (3a).

9. Électrode selon la revendication 8, **caractérisée en ce que** le premier matériau (3) électriquement conducteur est un métal ou un alliage métallique, par exemple un film synthétique conducteur en continu ou en surface à travers des fibres de carbone conductrices, ou un matériau textile conducteur en continu ou en surface.

10. Électrode selon la revendication 8 ou 9, **caractérisée en ce que** le deuxième matériau (3a) est formé d'une paire argent/chlorure d'argent ou étain/chlorure d'étain ou d'une autre paire redox adaptée à la dépolarisation de l'électrode.

11. Électrode selon l'une quelconque des revendications 1 à 10, **caractérisée en ce que** le support (1) ainsi qu'une éventuelle couche d'appui (6) y étant reliée présentent dans la zone à côté de l'élément de raccordement (5) au moins une incision (9) permettant une mobilité de l'élément de raccordement (5) par rapport à une couche de pansement (7) prévue pour être collée à la peau.

12. Électrode selon l'une quelconque des revendications 1 à 11, **caractérisée en ce que** l'élément de raccordement (5) est prémonté sur l'électrode et y est relié de manière permanente.

13. Procédé de fabrication d'une électrode à appliquer sur la peau humaine selon l'une quelconque des revendications 1 à 12, comprenant des étapes suivantes
- mise en place, de préférence par collage thermo-activé, d'un conducteur transversal en forme de bande sur la face inférieure tournée vers la peau d'un support électriquement non conducteur,
- réalisation, de préférence découpage d'une ouverture traversante à travers le conducteur transversal et le support,
- introduction dans l'ouverture d'un élément de raccordement monobloc à partir de la face inférieure du support, de telle manière qu'un point de raccordement pour un conducteur de signaux fait saillie sur la face supérieure opposée du support et que l'élément de raccordement repose sur le conducteur transversal avec une zone de maintien dépassant latéralement - de préférence en forme d'assiette,
- recouvrement de la zone de maintien de l'élément de raccordement d'une couche d'appui qui est collée au support latéralement à côté de la zone de maintien.

14. Procédé selon la revendication 13, **caractérisé par** des étapes supplémentaires suivantes :
- mise en place - de préférence par collage - d'une couche de pansement adhésif côté peau avec le support et/ou la couche d'appui,
- introduction d'un milieu de contact électrique - de préférence d'un gel - dans un évidement de la couche de pansement, de telle manière que le conducteur transversal sous-jacent est mis en contact.
